# EUROPÄISCHE PATENTSCHRIFT

(11) **EP 0 949 902 B1**
(45) Veröffentlichungstag und Bekanntmachung des Hinweises auf die Patenterteilung: **04.09.2002**
(21) Anmeldenummer: 97953637.2
(22) Anmeldetag: 12.12.1997
(51) Int. Cl.: A61K 7/48, A61K 7/06

(54) **KOSMETISCHES PRÄPARAT MIT PEPTIDZUSATZ**
COSMETIC PREPARATION WITH A PEPTIDE ADDITION
PREPARATION COSMETIQUE AVEC ADDITIF PEPTIDIQUE

(30) Priorität: 12.12.1996 DE 19653736
(43) Veröffentlichungstag der Anmeldung: 20.10.1999
(73) Patentinhaber: LANCASTER GROUP GmbH, 55116 Mainz (DE)
(72) Erfinder: GOLZ-BERNER, Karin, MC-98000 Monaco (MC); ZASTROW, Leonhard, MC-98000 Monaco (MC); DOMLOGE, Nouna, F-06190 Roquebrunne-Cap-Martin (FR)
(74) Vertreter: Walter, Wolf-Jürgen
(86) Internationale Anmeldenummer: DE9702941
(87) Internationale Veröffentlichungsnummer: WO98025584

(56) Entgegenhaltungen:
- EP-A- 0 297 457
- WO-A-95/08564
- DE-C- 4 241 154
- FR-A- 2 691 465

## Beschreibung

Die Erfindung betrifft ein neues kosmetisches Präparat, das von α-MSH ("Melanocyte Stimulating Hormone") abgeleitete Peptid-Derivate und weitere Wirkkomponenten enthält.

α-MSH ist bereits von einer Reihe von Forschungsgruppen untersucht worden, ohne daß bisher ein Arzneimittel daraus entwickelt werden konnte. Eine spezielle Wirkungsrichtung ist in der FR-A-2710340 offenbart worden, wo bestimmte von α-MSH abgeleitete Peptidderivate als für die Melanogenese stimulierende Wirkstoffe beschrieben und beansprucht wurden. Auch die topische Verwendung gegen entzündliche Reaktionen der Haut ist genannt.

Der Erfindung liegt die Aufgabe zugrunde, ein neues kosmetische Präparat mit insbesondere die Melanogenese fördernden und entzündungswidrigen Eigenschaften mit verbesserter Wirksamkeit zu entwickeln.

Erfindungsgemäß besteht ein kosmetisches Präparat mit Peptidzusatz aus einer Kombination der folgenden wirksamen Bestandteile:
a) ein Peptidderivat der Formel [Lip]X-His-Phe-Arg-Y in der Lip Thioctinsäure oder eines ihrer Derivate darstellt,
   - X: Glu, OH oder NH₂ bedeutet,
   - Y: Trp-Gly-OH,
   Trp-Gly-NH₂,
   Trp-NH₂ oder
   Trp-OH bedeutet,
   Phe Homo-Phe oder P-Fluor-Phe darstellt,
   und die Aminosäuren in der Form D, L und DL vorliegen können, oder Gemische davon, in einem Anteil von 0,05 bis 2,5 mg reines Peptidderivat pro kg Gesamtmasse, wobei das Peptidderivat mit Xanthin in einem Anteil von 0,5 bis 2 Mol pro 100 Mol Peptid assoziiert ist;
b) wenigstens 0,5 Gew-% eines Gemisches aus Enzymen und Vitaminen, das wenigstens 150 Einheiten/ml (U/ml) Superoxiddismutase enthält;
c) übliche Hilfs- und Trägerstoffe in einem Anteil von 65 bis 99,5 Gew.-%; und
d) weitere Wirkstoffe in einem Anteil von 0 bis 12 Gew-% enthält.

Die Prozentangaben sind jeweils auf die Gesamtmasse des kosmetischen Präparates bezogen.

Als Peptid wird ein aus der FR-A-2710340 bekanntes Lipoyl-Peptid oder Peptid-Gemisch eingesetzt, das insbesondere mindestens eine der folgenden Sequenzen umfaßt: I      [(DL)Lip]-Glu-His-D.HomoPhe-Arg-Trp-Gly-NH2 II [(DH)Lip]-Glu-His-D.HomoPhe-Arg-Trp-Gly-NH2 III [(DL)Lip]-Glu-His-paraFluorPhe-Arg-Trp-Gly-NH2 IV [(DH)Lip]-His-D.HomoPhe-Arg-Trp-Gly-NH2 V [N.Lipoyl-Lysin]-Glu-His-D.HomoPhe-Arg-Trp-Gly-NH2 VI    [N.Lipoyl-Lysin]-His-D.HomoPhe-Arg-Trp-Gly-NH2 VII [N.Lipoyl-Lysin]-His-D.HomoPhe-Arg-Trp-NH2 sowie die Derivate dieser Moleküle in Form von Salzen der Ester oder Amide, wobei das Peptid-Derivat mit Xanthin in einem Anteil von 0,5 bis 2 Mol pro 100 Mol Peptid versetzt ist.

In handelsüblichen Peptiden, z.B. in MAP® oder MAP-X® (von Laboratoties Seporga, Frankreich), einem Lipoylaminopeptid der obigen Peptid-Derivate, liegt der reine Peptidgehalt bei etwa 50 mg/kg, und davon werden ca. 0,01 bis 5 Gew-% in dem kosmetischen Präparat eingesetzt. Daraus ergibt sich der oben genannte Peptidgehalt für die Erfindung.

Das eingesetzte Gemisch aus Enzymen und Vitaminen ist vorzugsweise ein durch Ultraschallbehandlung hergestelltes Aufschlußprodukt einer Hefe, wobei das Aufschlußprodukt SOD, Protease, Vitamin B₂, Vitamin B₆, Vitamin B₁₂, Vitamin D₂ und Vitamin E enthält. Vorzugsweise enthält es wenigstens 150 U/ml SOD, Protease und die Vitamine B und D, wobei das Verhältnis SOD:Protease als internationale Einheiten wenigstens im Bereich von 3:1 bis 8:1 liegt.

Besonders vorteilhaft für die Herstellung des Enzym-/Vitamingemisches der Erfindung ist ein Aufschlußverfahren mittels Ultraschall, das in der DE 4241154C1 beschrieben ist und bei dem in einer Ultraschall-Durchflußzelle eine Zelldispersion oder Suspension durch einen Beschallungsraum geführt wird, bei dem die Sonotrode zur Hälfte bis Zweidrittel ihrer Länge in die Durchflußzelle hineinragt und in das zu beschallende Medium eintaucht. Dabei hat die Sonotrode einen Winkel von 80,5 bis 88,5°, und das Verhältnis der Eintauchlänge der Sonotrode in mm zum Beschallungsvolumen in ml wird auf einen Wert von 1:1,1 bis 1:20 eingestellt. Der Feststoffanteil in dem zu beschallenden Medium liegt im Bereich von 1:0,02 bis 1:2,2 (in Gew.-%).

Als Zelldispersion können Hefen, wie Bäckerhefe, Brauereihefe, Weinhefe sowie besonders behandelte Hefen, wie z.B. SODangereicherte Hefen, eingesetzt werden. Eine vorteilhaft einzusetzende Zelldispersion enthält z. B. Saccharomyces cerevisiae.

Ein besonders vorteilhafter Bestandteil a) des kosmetischen Präparates der Erfindung besteht aus einem Gemisch des mit Xanthin versetzten Peptidderivates der Formel

[Lip]X-His-Phe-Arg-Y

mit maritimen halbsynthetischen Peptiden und Polypeptiden, die eine biotechnologische Proteinfraktion darstellen, hergestellt aus Mikro-Algen der Gattung Chlorella und mit Byssus (Muschelseide) assoziierten Makro-Algen der Gattung Ulva und nachfolgender Assoziierung mit einem pflanzlichen Glucosepolymeren, und wobei die maritimen halbsynthetischen Peptide assoziiert sind mit 0,5 bis 5 Gew-% maritimen Mineralsalzen und Spurenelementen.

Ein solches Gemisch ist beispielsweise, das unter dem Namen "Sun Marine Complex" erhältliche Produkt (von Laboratories Seporge, Sophia-Antipolis Cedex, Frankreich).
Dieses vorteilhaft einzusetzende Gemisch aus Xanthin-assoziierten Peptiden und maritimen halbsynthetischen Peptiden, letztere assoziiert mit Glucosepolymeren, z.B. Dextrin, und Mineralsalzen/Spurenelementen aus Meerwasser, das nachfolgend als SMC (Sun Marine Complex) bezeichnet wird, wird durch einfaches Vermischen beider Komponenten hergestellt. Die Herstellung der maritimen halbsynthetischen Peptide erfolgt durch enzymatische Reinigung der tyrosinreichen Peptide und Polypeptide aus Byssus, d.h. der sog. Muschelseide von Meeresmuscheln. Danach wird enzymatisch der pflanzliche Zelluloseteil (Mikro- und Makro-Algen) behandelt, um die verschiedenen mineralischen und organischen Elemente zu extrahieren. Anschließend wird unter leicht basischen Bedingungen und unter relativ hohem Druck und erhöhter Temperatur die Assoziation mit einem Glucose-Polymeren durchgeführt, z.B. mit Dextrin, um eine Stabilisierung der Einzelelemente des Gemisches auf den glucosidischen Träger als "Transportmolekül" für den Organismus zu erreichen.

In dem SMC liegt der Anteil des mit Xanthin assoziierten Peptids im Bereich von 0,5 bis 10 Gew-%, vorzugsweise 0,5 bis 5 Gew-%. Den Rest nehmen die maritimen halbsynthetischen Peptide (assoziiert an z.B. Detrin) sowie Mineralsalze und Spurenelemente ein.

Als übliche Hilfs- und Trägerstoffe des erfindungsgemäßen topischen Präparates können verwendet werden z. B. Isopropylmyristat, Isopropylpalmitat, Isopropylstearat, Carbomer, Cetearylalkohol, Lecithin, Copolymere, Paraffinöl, Cetylalkohol, Propylenglycol, Polyglycol, Jojobaöl, Siliconöl, Kokosnußöl, Kaolin modifiziert gemäß WO95/17157, cetylpalmitat, Acrylates C10-C30 Alkyl Acrylate Crosspolymer, Magnesium Aluminium Silicate, Hydroxyethylcellulose sowie weitere, den speziellen Anwendungsformen wie z. B. Lippenstift, Augenkosmetik, Haarmaske usw. angepaßte Stoffe, die dem Fachmann bekannt sind.

Als weitere Wirkstoffe kann das Präparat enthalten 1.3 und 1.6-β-Glucan, CM-Glucan®, Allantoin, TiO₂, ZnO, UVA- und UVBblockierende Substanzen.

Weiterhin einsetzbar in der Emulsion mit einem Anteil von 0,1 bis 30 Gew-% sind auch neue Agglomerate von sphärischen unporösen SiO₂-Teilchen von 0,05-1,5 µm und sphärischen TiO₂- oder ZnO-Teilchen, wobei die Agglomerate eine Teilchengröße von 0,06-5 µm haben. Die Herstellung derartiger Agglomerate erfolgt durch Vermischen der Teilchen unter Rühren bei 300-400 U/Min und teilweisem Wasserzusatz bis zur pastenförmigen Konsistenz und anschließendem Restwasserzusatz und Homogenisierung bei 3000 bis 5000 U/Min für 20-60 Minuten.

Das erfindungsgemäße kosmetische Präparat zeigt überraschend eine wesentlich über das Potential der Einzelkomponenten hinausgehende synergistische entzündungswidrige Wirkung und kann daher erfolgreich in kosmetischen Zusammensetzungen, wie Sonnenschutzemulsionen, After-Sun-Emulsionen, Gesichtskosmetika usw. sowie bei sonstigen entzündlichen Prozessen der Haut kosmetisch eingesetzt werden. Besonders eindrucksvoll erweist sich dieser Synergismus darin, daß bereits eine Emulsion, die nur 0,5 Gew.-% des mit Xanthin assoziierten Peptidpräparates (mit 50 mg Peptid pro kg) neben den Hefeaufschlußprodukten enthält, die gleiche entzündungswidrige Wirkung zeigt, wie ein das reine Peptidpräparat (mit 50 mg Peptid/kg).

Das beweisen Messungen an Probanden mit einem Mexameter MX16® (Courage + Khazaka/Deutschland), bei dem der Rötungsgrad der Haut als Grundlage genommen wurde und in zeitlichen Abständen nach der Bestrahlung Messungen erfolgten. Die sich dabei ergebenden Kurven sowie die parallel dazu bei Probanden durchgeführten histologischen Hautuntersuchungen nach dem H+E Staining-Verfahren hinsichtlich der Melanocytenaktivität lassen erkennen, daß die Aktivität des erfindungsgemäßen Präparates weit über den zu erwartenden Werten der Aktivität der Einzelkomponenten liegt.

Eine bevorzugte Formulierung enthält die Peptidkomponente in einem Anteil von 0,05 bis 1,5 mg reines Peptidderivat pro kg Gesamtmasse.

Der erfindungsgemäße Bestandteil a) der Kombination von Xanthin-assoziierten Peptiden, Peptidderivaten und in der biotechnologischen Proteinfraktion aus maritimen halbsynthetischen Peptiden enthaltenen Enzymaktivatoren - letztere sind besonders reich an Tyrosin und Phenylalanin - wirkt stimulierend, schützend und regenerierend auf die Haut.

Die Hautstimulierung erfolgt durch den unter AMPc-Beteiligung ablaufenden Metabolismus, der durch Peptide und Xanthin aktiviert wird. Die Peptidderivate, insbesondere MAPX®, rufen eine Stimulierung der Melanin-Synthese hervor. Die halbsynthetischen Peptide regenerieren das Bindegewebe.

Die schützende und regenerierende Wirkung besteht einerseits in dem Lichtschutz, der durch neu synthetisiertes Melanin hervorgerufen wird, das die Rolle eines natürlichen UV-Filters spielt. Weiterhin besteht sie in der Regenerierung UV-geschädigter Zellen durch eine Modulierung der Cytokinine IL-1α und TNFα sowie eine synergistische Wirkung aller enthaltenen Peptide gegenüber freien Radikalen. Die entzündungswidrige Wirkung (anti-IL-1α und anti-TNFα) wurde experimentell mit 55 % bzw. 40 % nachgewiesen.

Der eingesetzte Hefeextrakt bewirkt unter anderem eine bessere Struktur der Hautzellen sowie einen verbesserten Feuchtigkeitshaushalt.

Dermatologische Untersuchungen der Haut, auf die eine vorteilhafte Zusammensetzung aus Xanthin-assoziierten Peptiden und maritimen halbsynthetischen Peptiden in Assoziation mit Glucosepolymeren und maritimen Mineralsalzen/Spurenelementen aufgetragen worden war, zeigten eine Verbesserung der Hautmorphologie dahingehend, daß das Keratin sehr gut hydratisiert war und wesentlich weniger Vakuolen und ödeme im Vergleich mit unbehandelten Hautpartien erkennbar waren. Die Dermis war gut konserviert mit einem normalen Bindegewebe. Der Gehalt an physikalischen und chemischen Lichtschutzfiltern gab weniger Hautirritationen nach vierundzwanzigstündiger Anwendung.

Die Verwendung des erfindungsgemäßen kosmetischen Präparates kann z.B. erfolgen in Sonnencremes, Sonnengelen, After-sun-Produkten, Tagescremes, Nachtcremes, Masken, Körperlotionen, Reinigungsmilch, Make up's, Lippenstiften, Körperpuder, Augenkosmetik, Haarmasken, Haarspülungen, Haarshampoos, Duschgelen, Duschölen, Badeölen. Die Herstellung derartiger Produkte erfolgt auf eine weise, wie sie dem Fachmann auf diesem Gebiet bekannt ist.

Die Erfindung soll nachstehend durch Beispiele näher erläutert werden. Die Prozentangaben in den Beispielen sind in Gewichtsprozent.

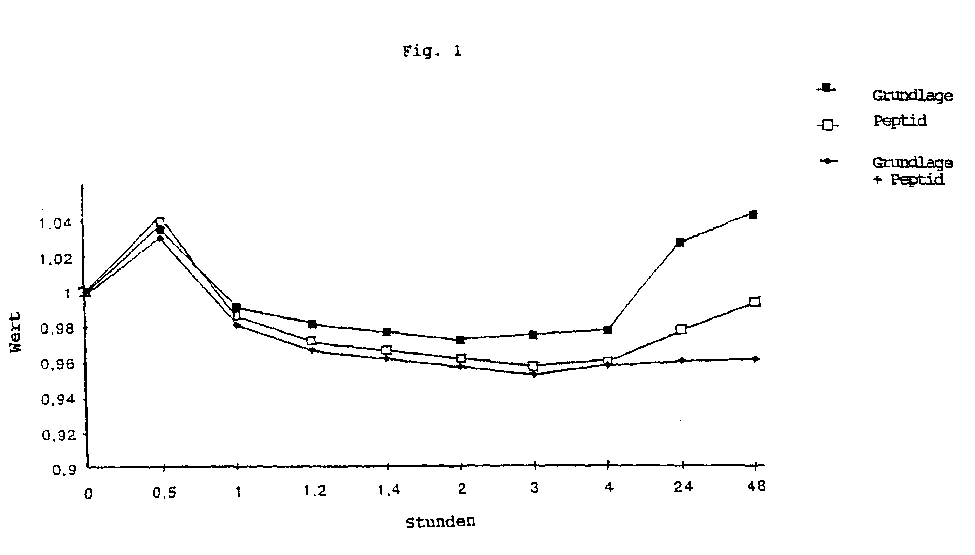

In der dazugehörigen Zeichnung zeigt Fig. 1 ein Diagramm der Mexameter-Messung geröteter Haut mit einem Vergleich verschiedener Emulsionen.

### Beispiel 1

### 1A) Herstellung des Hefeaufschlußproduktes

Es wurde eine Hefesuspension aus Superoxiddismutase angereicherter Hefe des Stammes Saccharomyces cerevisiae mit den folgenden Bestandteilen hergestellt:

| | |
|---|---|
| 20 % | Hefe |
| 10 % | Propylenglycol, |
| 0,4 % | Konservierungsmittel |
| ad 100 | Wasser. |

Das Vermischen erfolgte unter Kühlung bei etwa 8 bis 10 °C. Die Hefesuspension wurde gemäß Beispiel 1 der DE 4241154C1 behandelt, wobei bei einer maximalen Temperatur von 20° C zirka 3 1 Aufschlußprodukt pro Stunde aus der Durchflußzelle abgezogen werden konnten. Nach der Abtrennung der Zellwände erhielt man ein Produkt mit folgenden Gehalten an aktiver Substanz

| | |
|---|---|
| SOD ≥ 200 U/ml | Vitamin B₆ 40 mg/l |
| Protease ~ 50 U/ml | Vitamin B₁₂ 3 mg/l |
| Vitamin B₂ 20 mg/l | Vitamin D₂ 0,3 mg/ml |
| Vitamin E 0,6 mg/ml. | (U = Einheiten) |

### 1B) Herstellung der kosmetischen Emulsion

Es wurde in den folgenden Beispielen mit dem Hefeaufschlußprodukt nach Beispiel 1A gearbeitet und mit der folgenden allgemeinen Arbeitsvorschrift in den Beispielen 2 - 4.

Zur Phase A erfolgte bei ca. 80 °C und unter Rühren die Zugabe der ebenfalls auf 80 °C erwärmten Phase B. Das Gemisch wurde homogenisiert, abgekühlt und bei etwa 35 °C mit der Phase C versetzt. Das Gemisch wurde homogenisiert.

### Beispiel 2 Sonnencreme

Die Herstellung erfolgte gemäß Beispiel 1B.

| **Phase A** | |
|---|---|
| Glycerin | 3,0 % |
| Magnesiumsulfat | 0,5 % |
| Wasser | ad 100 |

| **Phase B** | |
|---|---|
| Glyceryl Oleate | 2,5 % |
| Decyl Oleate | 5,0 % |
| Paraffinoil | 10,0 % |
| Bienenwachs | 2,0 % |
| TiO₂ | 3,0 % |
| Zinkstearat | 2,0 % |

| **Phase C** | |
|---|---|
| Konservierungsmittel | 0,3 % |
| Parfümöl | 0,5 % |
| Peptidpräparat MAP-X® | 1,0 % |
| Hefeaufschlußprodukt gemäß Bsp.1A | 0,5 % |

### Beispiel 3 After-Sun-Präparat

Die Herstellung erfolgte gemäß Beispiel 1B.

| **Phase A** | |
|---|---|
| Glycerin | 6,0 % |
| Magnesiumsulfat | 1,0 % |
| Wasser | ad 100 |

| **Phase B** | |
|---|---|
| Glyceryl Oleate | 4,0 % |
| Polyglyceryl-3 Diisostearate | 10,0 % |
| Hexyldecanol | 2,0 % |
| Bienenwachs | 1,0 % |
| Dicapryl Ether | 2,0 % |

| **Phase C** | |
|---|---|
| Konservierungsmittel | 0,4 % |
| Parfümöl | 0,5 % |
| Peptidpräparat MAP-X® | 2,0 % |
| Hefeaufschlußprodukt gemäß Bsp.1A | 5,0 % |

### Beispiel 4 Körperlotion

Die Herstellung erfolgte gemäß Beispiel 1B.

| **Phase A** | |
|---|---|
| Glycerin | 3,0 % |
| Magnesiumsulfat | 0,5 % |
| Propylenglycol | 2,0 % |
| Wasser | ad 100 |

| **Phase B** | |
|---|---|
| Glyceryl Oleate | 1,0 % |
| Polyglyceryl-2 Dipolyhydroxy- | |
| stearate | 2,5 % |
| Cetearyl Jonanoate | 3,0 % |
| Jojobaöl | 1,5 % |

| **Phase C** | |
|---|---|
| Konservierungsmittel | 0,4 % |
| Parfümöl | 1,5 % |
| Peptidpräparat MAP-X® | 3,0 % |
| Hefeaufschlußprodukt gemäß Bsp.1A | 8,0 % |

### Beispiel 5 Lippenstift

Die Phase A wurde bei ca. 85 °C geschmolzen. Die Phase B wurde mit der Farbe verrührt, und es wurde die Phase A zugegeben. Anschließend erfolgte die Zugabe der Phase C unter Rühren, und es wurde abgekühlt.

| **Phase A** | |
|---|---|
| Candelillawachs | 8,0 % |
| Bienenwachs | ad 100 |

| **Phase B** | |
|---|---|
| Ceraphyloil | 25,0 % |
| Calendulaoil | 25,0 % |
| Farbe (Color; je nach Farbe) | 8,0 % |

| **Phase C** | |
|---|---|
| Peptidpräparat MAP-X® | 2,0 % |
| Hefeaufschlußprodukt gemäß Bsp.1A | 2,0 % |
| Aroma | 1,0 % |

### Beispiel 6 Duschgel

In das vorgelegt Wasser wurden die nachfolgenden Bestandteile in üblicher Weise unter Rühren und anschließendem Homogenisieren eingebracht.

| | |
|---|---|
| Wasser | ad 100 |
| Sodium Myreth Sulfate | 25 % |
| Disodium Laurethsulfosuccinate | 8 % |
| Konservierungsmittel | 0,2 % |
| Parfümöl | 0,5 % |
| Peptidpräparat MAP-X® | 0,5 % |
| Hefeaufschlußprodukt gemäß Bsp.1A | 0,1 % |

### Beispiel 7

In Fig. 1 ist ein Vergleich verschiedener Emulsionen bei der Messung geröteter Haut von Probanden dargestellt. Die Werte sind Mittelwerte von 10 Messungen. Der Anfangswert 1 wurde als dimensionslose Verhältniszahl (Index) willkürlich gesetzt. Die Messungen erfolgen zu den in den Kurven angegebenen Zeiten. Die Messung erfolgte mit einem Mexameter MX16® der Firma Courage + Khazaka electronic GmbH, Deutschland. Gemessen wird dabei die Absorption der Wellenlängen 568 und 660 nm. Dabei entspricht die eine Wellenlänge etwa den Hautabsorptionsspitzen von Hämoglobin, die andere schließt andere Farbeinflüsse (z.B. Bilirubin) möglichst aus. Die erzielten Meßwerte werden als Verhältniszahl zwischen den benutzten Farben (Index) angegeben.

Verglichen wurde
A eine Basisemulsion, die nur die Emulsionsgrundlage und 0,5 Gew-% des Hefeaufschlußproduktes gemäß Beispiel 1A enthielt ("Grundlage"),
B das Peptidpräparat MAP-X® in der Konzentration 50 mg reines Peptid pro kg ("Peptid"),
C die gleiche Basisemulsion wie bei A mit 0,5 % Peptidpräparat MAP-X® ("Peptid+Grundlage").

Der Kurvenverlauf nach 24 bzw. 48 Stunden zeigt deutlich, daß die Emulsion C eine nicht zu erwartende Verbesserung im Verhältnis zu A oder B zeigt.

Da die Wirkung von Antioxidantien in der Art von komplexen physiologischen Regelkreisen in einem biologischen System bewertet werden muß, ist ein einfacher Additionseffekt bei zwei Substanzen mit jeweils anti-oxidativer Wirkung nicht zu beobachten. Stattdessen ist nur eine gering über dem Normwert liegende Gesamtwirkung zu erwarten. Somit ist der Unterschied in den vorliegenden Meßergebnissen klar als Synergismus zu bewerten.

### Beispiel 8 Hautschutzcreme für 24 h (Vor- und Nach-Sonnenschutz)

| **Phase A** | |
|---|---|
| C12-15 Alkyl Benoate | 4,0 % |
| Sheabutter | 2,0 % |
| Steareth-2 | 1,5 % |

| **Phase B** | |
|---|---|
| destilliertes Wasser | ad 100 |
| Cross Polymer | 0,5 % |
| Glycerine | 2 % |

| **Phase C** | |
|---|---|
| Triethanolamine | 0,5 % |

| **Phase D** | |
|---|---|
| Jojobaöl | 2 % |
| Olivenöl | 1 % |
| Konservierungsmittel | 0,5 % |

| **Phase E** | |
|---|---|
| Sun Marine Complex | 5,0 % |
| Hefeaufschlußprodukt gemäß Beispiel 1B | 0,5 % |
| Parfümöl | 0,2 %. |

Die Herstellung erfolgte in der Weise, daß die Bestandteile der Phasen A und B jeweils getrennt bei etwa 60° C verrührt wurden und anschließend beide Phasen bei dieser Temperatur miteinander vermischt und auf cirka 45° C abgekühlt wurden. Danach wurden die Phasen C und D hinzugegeben, und es wurde unter Rühren abgekühlt. Die Phase E wurde bei 35° C hinzugesetzt und mit dem Gesamtgemisch verrührt.

### Beispiel 9 Sonnencreme (SPF10)

| **Phase A** | |
|---|---|
| Steareth-2 | 3 % |
| Steareth-21 | 2 % |
| Bienenwachs | 1,5 % |

| **Phase B** | |
|---|---|
| destilliertes wasser | ad 100 |
| Glycerine | 3,5 % |
| Propylen glycole | 2,0 % |
| TiO₂ | 7,0 % |

| **Phase C** | |
|---|---|
| Jojobaöl | 2,5 % |
| Babassuöl | 1,0 % |
| Siliconöl | 0,5 % |
| Konservierungsmittel | 0,3 % |

| **Phase D** | |
|---|---|
| Sun Marine Complex | 0,5 % |
| Hefeaufschlußprodukt gemäß Anspruch 1B | 0,5 % |
| Parfümöl | 0,1 % |

Die Verarbeitung erfolgte wie im Beispiel 8.

### Beispiel 10 Sonnencreme (SPF15)

| **Phase A** | |
|---|---|
| Steareth-2 | 2,0 % |
| Steareth-21 | 2,0 % |
| Isohexadecane | 3,0 % |
| Octyl Methoxycinnamate | 5,0 % |
| 4-Methylbenzylidene camphor | 3,3 % |

| **Phase B** | |
|---|---|
| destilliertes Wasser | ad 100 |
| Glycerine | 10,0 % |
| TiO₂/SiO₂-Agglomerate* | 2,0 % |
| ZnO/SiO₂-Agglomerate* | 1,0 % |

| **Phase C** | |
|---|---|
| Siliconöl | 2,0 % |
| Palmöl | 4,0 % |
| Konservierungsmittel | 0,3 % |

| **Phase D** | |
|---|---|
| Sun Marine Complex | 0,5 % |
| Hefeaufschlußprodukt gemäß Beispiel 1B | 0,5 % |
| Parfümöl | 0,2 % |
| Die Verarbeitung erfolgte gemäß Beispiel 8. | |

| | |
|---|---|
| * aus sphärischen unporösen SiO₂-Teilchen von 0,05-1,5 µm und sphärischen TiO₂- oder ZnO-Teilchen, wobei die Agglomerate eine Teilchengröße von 0,06-1,5 µm haben. | |

### Beispiel 11 Sonnencreme mit chemischen Filtern

| **Phase A** | |
|---|---|
| Cetearyl | 1,5 % |
| Glyceryl Stearathe and Ceteareth 20 and Cetyl | |
| Palmitate | 3,5 % |
| Octyl Stearathe | 1,5 % |
| Octyl Methoxycinnamate | 6,5 % |
| 4-Methylbenzylidene Camphor | 1,5 % |

| **Phase B** | |
|---|---|
| destilliertes Wasser | ad 100 |
| Glycerine | 2,0 % |

| **Phase C** | |
|---|---|
| Babassuöl | 5,0 % |
| Konservierungsmittel | 0,5 % |

| **Phase D** | |
|---|---|
| Sun Marine Complex | 3,0 % |
| Hefeaufschlußprodukt gemäß Beispiel 1B | 0,5 % |
| Parfümöl | 0,1 % |

Die Verarbeitung der Zusammensetzung erfolgte gemäß Beispiel 8.

### Beispiel 12 Make-up mit SPF4

| **Phase A** | |
|---|---|
| Sheabutter | 2 % |
| Bienenwachs | 3 % |
| Olivenöl | 5 % |
| Farbe | 3-10 % |
| TiO₂ | 4 % |

| **Phase B** | |
|---|---|
| destilliertes Wasser | ad 100 |
| Glycerin | 2 % |

| **Phase C** | |
|---|---|
| Jojobaöl | 2 % |
| Siliconöl | 5 % |

Die Verarbeitung der Zusammensetzung erfolgte gemäß Beispiel 8.

## Patentansprüche

1. Kosmetisches Präparat mit Peptidzusatz, **dadurch gekennzeichnet, daß** es als wirksame Bestandteile eine Kombination der folgenden Bestandteile enthält:
a) ein Peptidderivat der Formel [Lip]X-His-Phe-Arg-Y in der Lip Thioctinsäure oder eines ihrer Derivate darstellt,
X Glu, OH oder NH₂ bedeutet,
Y Trp-Gly-OH,
Trp-Gly-NH₂,
Trp-NH₂ oder
Trp-OH bedeutet,
Phe Homo-Phe oder P-Fluor-Phe darstellt, und die Aminosäuren in der Form D, L und DL vorliegen können, oder Gemische davon, in einem Anteil von 0,05 bis 2,5 mg reines Peptidderivat pro kg Gesamtmasse, wobei das Peptidderivat mit Xanthin in einem Anteil von 0,5 bis 2 Mol pro 100 Mol Peptid versetzt ist;
b) wenigstens 0,5 Gew-% eines Gemisches aus Enzymen und Vitaminen, das wenigstens 150 U/ml Superoxiddismutase (SOD) enthält;
c) übliche Hilfs- und Trägerstoffe in einem Anteil von 65 bis 99,5 Gew.-%; und
d) 0 bis 12 Gew-% weitere Wirkstoffe enthält;
wobei die Prozentangaben jeweils auf die Gesamtmasse des Präparates bezogen sind.

2. Präparat nach Anspruch 1, **dadurch gekennzeichnet, daß** das Gemisch aus Enzymen und Vitaminen aus SOD, Protease, Vitamin B₂, Vitamin B₆, Vitamin B₁₂, Vitamin D₂ und Vitamin E besteht.

3. Präparat nach Anspruch 1 oder 2, **dadurch gekennzeichnet, daß** es Protease und die Vitamine B und D enthält, wobei das Verhältnis SOD:Protease, ausgedrückt als internationale Einheiten, wenigstens im Bereich von 3:1 bis 8:1 liegt.

4. Präparat nach Anspruch 2, **dadurch gekennzeichnet, daß** das Enzym- und Vitamingemisch aus dem Ultraschallaufschluß einer Hefe herrührt, insbesondere aus dem Aufschluß von Bäckerhefe.

5. Präparat nach Anspruch 1, **dadurch gekennzeichnet, daß** das Peptid ausgewählt ist unter den Peptiden oder Peptidgemischen, die mindestens eine der folgenden Sequenzen umfassen: I      [(DL)Lip]-Glu-His-D.HomoPhe-Arg-Trp-Gly-NH2 II [(DH)Lip]-Glu-His-D.HomoPhe-Arg-Trp-Gly-NH2 III [(DL)Lip]-Glu-His-paraFluorPhe-Arg-Trp-Gly-NH2 IV [(DH)Lip]-His-D.HomoPhe-Arg-Trp-Gly-NH2 V [N.Lipoyl-Lysin]-Glu-His-D.HomoPhe-Arg-Trp-Gly-NH2 VI [N.Lipoyl-Lysin]-His-D.HomoPhe-Arg-Trp-Gly-NH2 VII [N.Lipoyl-Lysin]-His-D.HomoPhe-Arg-Trp-NH2 sowie die Derivate dieser Moleküle in Form von Salzen der Ester oder Amide.

6. Präparat nach einem der Ansprüche 1 bis 5, **dadurch gekennzeichnet, daß** die Peptidkomponente in einem Anteil von 0,05 bis 1,5 mg reines Peptidderivat pro kg Gesamtmasse enthalten ist.

7. Präparat nach Anspruch 1, **dadurch gekennzeichnet, daß** die Peptidkomponente ein Lipoylaminopeptid ist, dessen reiner Peptidgehalt bei 50 mg/kg liegt, und wobei die Komponente mit 0,01 bis 5 Gew-% in dem kosmetischen Präparat enthalten ist.

8. Präparat nach einem der Ansprüche 1 bis 8, **dadurch gekennzeichnet, daß** das mit Xanthin versetzte Peptidderivat als Gemisch vorliegt von Peptidderivaten der Formel
[Lip]X-His-Phe-Arg-Y gemäß Anspruch 1 und maritimen halbsynthetischen Peptiden und Polypeptiden, die eine biotechnologische Proteinfraktion darstellen, hergestellt aus Mikro-Algen der Gattung Chlorella und mit Byssus (Muschelseide) assoziierten Makro-Algen der Gattung Ulva und nachfolgender Assoziierung mit einem pflanzlichen Glucosepolymeren, und wobei die maritimen halbsynthetischen Peptide assoziiert sind mit 0,5 bis 5 Gew-% maritimen Mineralsalzen und Spurenelementen.

9. Kosmetische Verwendung eines kosmetischen Präparates mit Peptidzusatz nach einem der Ansprüche 1 bis 8 als Sonnencreme, Sonnenemulsion, After-Sun-Lotion, Pre-Sun-Lotion, Körperlotion, Lippenstift, Make-up, Augenkosmetik, Gesichtspuder, Haarmaske, Haarshampoo, Haarwasser, Duschgel, Duschöl.

## Claims

1. A cosmetic preparation with a peptide additive, comprising as active ingredients a combination of the following ingredients:
a) a peptide derivative of the formula [Lip]X-His-Phe-Arg-Y, where Lip represents thioctic acid or one of its derivatives,
X denotes Glu, OH, or NH₂
Y denotes Trp-Gly-OH.
Trp-GlY-NH₂,
Trp-NH₂ or
Trp-OH,
Phe denotes Homo-Phe or P-fluoro-Phe, and the amino acids may be present in the form D, L or DL, or mixtures thereof, in the amount of 0.05 to 2.5 mg pure peptide derivative per kg total weight, where the peptide derivative is mixed with xanthine in a ratio of 0.5 to 2 mol per 100 mol peptide;
b) at least 0,5 wt% of a mixture of enzymes and vitamins containing at least 150 U/mL peroxide dismutase (POD);
c) the usual additives and vehicles in the amount of 65 to 99.5 wt%; and
d) 0 to 12 wt% other active ingredients,
where the percentage amounts are all based on the total weight of the preparation.

2. A preparation according to Claim 1, wherein the mixture consists of enzymes and vitamins, peroxide dismutase, protease, vitamin B2, vitamin B6, vitamin B12, and vitamin E.

3. A preparation according to Claim 1 or 2, wherein it contains protease and vitamins B and D, with the ratio of peroxide dismutase to protease, expressed as international units, being at least in the range of 3:1 to 8:1.

4. A preparation according to Claim 2, wherein the enzyme and vitamin mixture originates from ultrasonic digestion of a yeast, especially from digestion of baker's yeast.

5. A preparation according to Claim 1, wherein the peptide is selected from peptides or peptide mixtures including at least one of the following sequences: I      [(DL)Lip]-Glu-His-D.HomoPHe-Arg-Trp-Gly-NH2 II [(DH)Lip]-Glu-His-D.HomoPhe-Arg-Trp-Gly-NH2 III [(DL)Lip]-Glu-His-para-fluoro-Phe-Arg-Trp-Gly-NH2 IV [(DH)Lip]-His-D.HomoPhe-Arg-Trp-Gly-NH2 V [N.Lipoyl-lysine]-Glu-His-D.HomoPhe-Arg-Trp-Gly-NH2 VI [N.Lipoyl-lysine]-His-D.HomoPhe-Arg-Trp-Gly-NH2 VII [N.Lipoyl-lysine]-His-D.HomoPhe-Arg-Trp-NH2 as well as derivatives of these molecules in the form of salts of the esters or amides.

6. A preparation according to one of Claims 1 through 5, wherein the peptide component is present in the amount of 0.05 to 1.5 mg pure peptide derivative per kg total weight.

7. A preparation according to one of Claims 1 through 6, wherein the peptide component is a lipoylaminopeptide, the pure peptide content is about 50 mg/kg, and 0.01 to 5 wt% of the component is contained in the cosmetic preparation.

8. A preparation according to one of Claims 1 through 8, wherein the peptide derivative mixed with xanthine is present in the form of a mixture of peptide derivatives of the formula [Lip]X-His-Phe-Arg-Y according to Claim 1 and semisynthetic marine peptides and polypeptides, which are a bioengineered protein fraction produced from microalgae of the genus Chlorella and macroalgae of the genus Ulva associated with byssus (mollusk silk), then associated with a vegetable glucose polymer, and wherein the semisynthetic marine peptides are associated with 0.5 to 5 wt% marine mineral salts and trace elements.

9. Cosmetic use of a cosmetic preparation with a peptide additive according to one of Claims 1 through 8 as a sun cream, sun emulsion, after-sun lotion, before-sun lotion, body lotion, lipstick, make-up, eye cosmetics, face powder, hair mask, hair shampoo, hair lotion, shower gel or shower oil.

## Revendications

1. Préparation cosmétique avec un ajout de peptide, **caractérisée en ce qu'**elle contient à titre de composants actifs une combinaison des composants efficaces suivants :
a) un dérivé de peptide de formule [Lip]X-His-Phe-Arg-Y, dans laquelle Lip représente l'acide thioctinique ou un de ses dérivés,
X représente Glu, OH ou MH₂,
Y représente Trp-Gly-OH,
Trp-Gly-NH₂,
Trp-NH₂, ou
Trp-OH,
Phe représente Homo-Phe ou P-fluor-Phe,
et les acides aminés peuvent se présenter sous forme D, L et DL, ou des mélanges de ceux-ci, en une fraction de 0,05 à 2,5 mg de dérivé peptidique pur par kg de masse totale, le dérivé peptidique étant associé à la xanthine en une fraction de 0,5 à 2 moles pour 100 moles de peptide ;
b) au moins 0,5 % en poids d'un mélange d'enzymes et de vitamines contenant au moins 150 unités/ml (U/ml) de superoxyde dismutase ;
c) des auxiliaires et véhicules, usuels en une fraction de 65 à 99,5 % en poids ; et
d) d'autres substances actives en une fraction de 0 à 12 % en poids ;
les indications de pourcentage étant respectivement rapportées sur la masse totale de la préparation.

2. Préparation selon la revendication 1, **caractérisée en ce que** le mélange d'enzymes et de vitamines se compose de SOD, de protéase, de vitamine B₂, vitamine B₆, vitamine B₁₂, vitamine D₂ et vitamine E.

3. Préparation selon la revendication 1 ou 2, **caractérisée en ce qu'**elle contient de la protéase et des vitamines B et D, le rapport SOD : protéase se situant, en unités internationales, au moins dans la plage allant de 3 : 1 à 8 : 1.

4. Préparation selon la revendication 2, **caractérisée en ce qu'**elle provient d'un mélange d'enzymes et de vitamines issu du traitement aux ultrasons d'une levure, notamment du traitement de la levure de boulanger.

5. Préparation selon la revendication 1, **caractérisée en ce que** le peptide est choisi parmi les peptides ou des mélanges de peptides comprenant en particulier au moins l'une des séquences suivantes : I    [(DL)Lip]-Glu-His-D.HomoPhe-Arg-Trp-Gly-NH2 II [(DH)Lip]-Glu-His-D.HomoPhe-Arg-Trp-Gly-NH2 III [(DL)Lip]-Glu-His-paraFluorPhe-Arg-Trp-Gly-NH2 IV [(DH)Lip]-His-D.HomoPhe-Arg-Trp-Gly-NH₂ V [N.Lipoyl-Lysin]-Glu-His-D.HomoPhe-Arg-Trp-Gly-NH2 VI [N.Lipoyl-Lysin]-His-D.HomoPhe-Arg-Trp-Gly-NH2 VII [N.Lipoyl-Lysin]-D.HomoPhe-Arg-Trp-NH2 ainsi que des dérivés de cette molécule sous forme de sels d'esters ou d'amides.

6. Préparation selon l'une des revendications 1 à 5,
**caractérisée en ce que** le composant peptidique est contenu en une fraction de 0,05 à 1,5 mg de dérivé peptidique pur par kg de masse totale.

7. Préparation selon la revendication 1, **caractérisée en ce que** le composant peptidique est un aminopeptide lipolyl dont la teneur en peptide pur se situe à environ 50 mg/kg, le composant étant contenu dans la préparation cosmétique entre 0,01 et 5 % en poids.

8. Préparation selon l'une des revendications 1 à 8, **caractérisée en ce que** le dérivé peptidique mélangé à la xanthine se présente sous forme de dérivés peptidiques de formule
[Lip]X-His-Phe-Arg-Y selon la revendication 1 de peptides et polypeptides semi-synthétiques maritimes, qui représentent une fraction protéique issue des biotechnologies, préparée à partir de micro-algues du genre Chlorella et avec des macro-algues du genre Ulva associées au Byssus (filaments soyeux de mollusques) et par association consécutive de polymères de glucose végétaux, les peptides semi-synthétiques maritimes étant associés à 0,5 à 5 % de sels minéraux et d'éléments traces maritimes.

9. Utilisation cosmétique d'une préparation cosmétique selon l'invention avec ajout de peptides selon l'une des revendications 1 à 8 comme crèmes solaires, émulsions solaires, lotions après-soleil, lotions avant-soleil, lotions corporelles, rouges à lèvres, maquillage, cosmétiques pour les yeux, poudres pour le visage, masques capillaires, shampooings, après-shampooings, gels douche, huiles de douche.
